# EUROPEAN PATENT APPLICATION

(11) **EP 3 015 458 A1**
(43) Date of publication of application: **04.05.2016**
(21) Application number: 14191501.7
(22) Date of filing: 03.11.2014
(51) Int. Cl.: C07D 231/12, C07B 37/10

(54) **Process for preparing 3,5-bis(haloalkyl)pyrazole derivatives from a,a-dihaloamines and ketimines**

(71) Applicant: Bayer CropScience AG, 40789 Monheim am Rhein (DE)
(72) Inventor: The designation of the inventor has not yet been filed
(74) Representative: BIP Patents

(57) **Abstract**

The present invention relates to a novel process for preparing 3,5-bis(haloalkyl)pyrazole derivatives from α,α-dihaloamines and ketimines.

## Description

The present invention relates to a novel process for preparing 3,5-bis(haloalkyl)pyrazole derivatives from α,α-dihaloamines and ketimines.

Polyfluoroalkylpyrazolylcarboxylic acid derivatives and 3,5-bis(haloalkyl)pyrazoles are valuable precursors of active fungicidal ingredients (WO 2003/070705, WO 2008/013925, WO 2012/025557).

Pyrazolecarboxylic acid derivatives are typically prepared by reacting acrylic acid derivatives having two leaving groups with hydrazines (WO 2009/112157 and WO 2009/106230). WO 2005/042468 discloses a process for preparing 2-dihaloacyl-3-aminoactylic esters by reacting acid halides with dialkylaminoacrylic esters and subsequent cyclization thereof with alkyl hydrazines. WO 2008/022777 describes a process for preparing 3-dihalomethylpyrazole-4-carboxylic acid derivatives by reacting α,α-difluoroamines in the presence of Lewis acids with acrylic acid derivatives and subsequent reaction thereof with alkylhydrazines.

3,5-bis(fluoroalkyl)pyrazoles are prepared by reacting bisperfluoroalkyl diketones (e.g. 1,1,1,5,5,5-hexafluoroacetylacetone) with hydrazines (cf. Pashkevich et al., Zhurnal Vsesoyuznogo Khimicheskogo Obshchestva im. D. I. Mendeleeva (1981), 26(1), 105-7), the yield being only 27 - 40%. The synthesis, isolation and purification of the polyfluoroalkyl diketones is very complex since the compounds are generally very volatile and highly toxic.

In the light of the prior art described above, it is an object of the present invention to provide a process that does not have the aforementioned disadvantages and hence gives a route to 3,5-bis(haloalkyl)pyrazole derivatives in high yields.

The object described above was achieved by a process for preparing 3,5-bis(haloalkyl)pyrazoles of the formula **(Ia)** and **(Ib),** 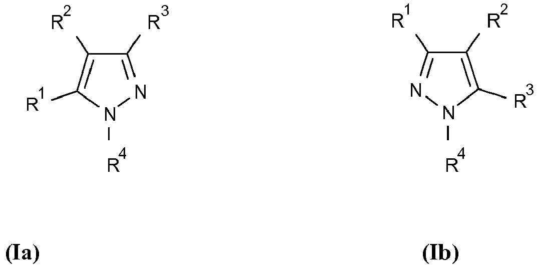 in which
- R¹ and R³: are each independently selected from C₁-C₆-haloalkyl;
- R²: is selected from H, halogen, COOH, (C=O)OR⁵, CN and (C=O)NR⁶R⁷;
- R⁴: is selected from H, C₁-C₈-alkyl, CH₂COOC₁-C₈-alkyl, aryl, pyridyl;
- R⁵: is selected from C₁-C₁₂-alkyl, C₃-C₈-cycloalkyl, C₆-C₁₈-aryl, C₇-C₁₉-arylalkyl and C₇-C₁₉-alkylaryl;
- R⁶ and R⁷: are each independently selected from C₁-C₁₂-alkyl, C₃-C₈-cycloalkyl, C₆-C₁₈-aryl, C₇-C₁₉-arylalkyl and C₇-C₁₉-alkylaryl or where
- R⁶ and R⁷: together with the nitrogen atom to which they are bonded may form a four-, five- or six-membered ring
characterized in that in step (A), α,α-dihaloamines of the formula **(II),** 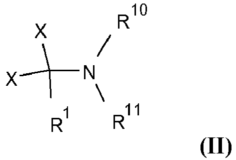 in which
- R¹: is as defined above;
- X: is independently selected from F, Cl or Br,
- R¹⁰ and R¹¹: are each independently selected from C₁-C₁₂-alkyl, C₃-C₈-cycloalkyl, C₆-C₁₈-aryl, C₇-C₁₉-arylalkyl and C₇-C₁₉-alkylaryl or where
- R¹⁰ and R¹¹: together with the nitrogen atom to which they are bonded may form a five- or six-membered ring;
are reacted with compounds of the formula **(III),** 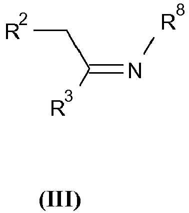 in which
- R⁸: is selected from C₁-C₁₂-alkyl, C₃-C₈-cycloalkyl, C₆-C₁₈-aryl, C₇-C₁₉-arylalkyl and C₇-C₁₉-alkylaryl, OR⁹;
- R⁹: is selected from C₁-C₁₂-alkyl, C₃-C₈-cycloalkyl, C₆-C₁₈-aryl, C₇-C₁₉-arylalkyl, C₇-C₁₉-alkylaryl;
- R² and R³: are as defined above;
to form the compound of formula **(V): (V**-**1)**, **(V-2), (V-3), (V-4) and (V-5)**

| |
|---|
| 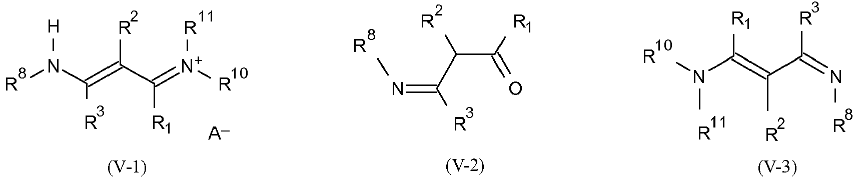 |
| 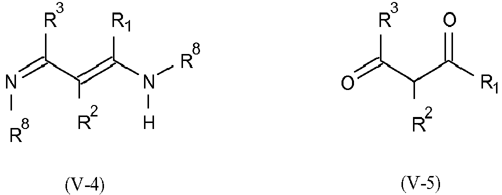 |
| A- is BF₄⁻, AlCl₃F⁻, AlF₂Cl₂⁻, AlF₃Cl⁻ or ZnCl₂F⁻ |
| R¹, R², R³, R⁵, R⁶, R⁷, R⁸, R⁹, R¹⁰, R¹¹ are as defined above |

and that in step (B) in the presence of hydrazine H₂N-NHR⁴ **(IV) -** with R⁴ being as defined above - a cyclization of **(V)** takes place to form **(Ia**/**Ib)**.

*Preferred* is a process according to the invention, where the radicals in formula **(Ia), (Ib), (II), (III), (IV)** and **(V)** are defined as follows:
- R¹ and R³: are each independently selected from difluoromethyl, trifluoromethyl, chlorofluoromethyl, dichlorofluoromethyl, chlorodifluoromethyl, 1-fluoroethyl, 2-fluoroethyl, 2,2-difluoroethyl, 2,2,2-trifluoroethyl, 2-chloro-2-fluoroethyl, 2-difluoroethyl, 2,2-dichloro-2-fluoroethyl, 2,2,2-trichloroethyl, tetrafluoroethyl (CF₃CFH), pentafluoroethyl and 1,1,1-trifluoroprop-2-yl;
- R²: is selected from H, F, Cl, Br, COOCH₃, COOC₂H₅, COOC₃H₇, CN and CON(CH₃)₂, CON(C₂H₅)₂;
- R⁴: is selected from H, C₁-C₈-alkyl, CH₂COOC₁-C₈-alkyl, phenyl, pyridyl;
- R⁸: are each independently selected from methyl, ethyl, *n-, iso*-propyl, *n-, iso-, sec-* and *t*-butyl, cyclopropyl, cyclobutyl, cyclopentyl, cyclohexyl, phenyl, benzyl, phenylethyl, C₇-C₁₉-alkylaryl, tolyl, 2,3-, 2,4-, 2,5-, 2,6-, 3,4- or 3,5-dimethylphenyl;
- X: is independently selected from F or Cl;
- R¹⁰ and R¹¹: are each independently selected from C₁-C₁₂-alkyl, C₃-C₈-cycloalkyl, C₇-C₁₉-arylalkyl or
- R¹⁰ and R¹¹: together with the nitrogen atom to which they are bonded may form a five- membered ring.

*Mote preferred* is a process according to the invention, where the radicals in formula **(Ia), (Ib), (II), (III), (IV)** and **(V)** are defined as follows:
- R¹ and R³: are each independently selected from trifluoromethyl, difluoromethyl, difluorochloromethyl, pentafluoroethyl;
- R²: is selected from H, Cl, CN, COOC₂H₅;
- R⁴: is selected from H, methyl, ethyl, *n-,* isopropyl, *n-, iso-, sec-* and *t*-butyl, *n*-pentyl, *n*-hexyl, 1,3-dimethylbutyl, 3,3-dimethylbutyl, phenyl, CH₂COOCH₃, CH₂COOCH₂CH₃;
- R⁸: is selected from methyl, ethyl, *n-, iso*-propyl, *n-, iso-, sec-* und *t*-butyl, cyclopropyl, cyclobutyl, cyclopentyl, cyclohexyl, benzyl, C₇-C₁₉-alkylaryl;
- X: is independently selected from F or Cl;
- R¹⁰ and R¹¹: are each independently selected from C₁-C₁₂-alkyl, C₃-C₈-cycloalkyl.

*Even more preferred* is a process according to the invention, where the radicals in formula **(Ia), (Ib), (II), (III), (IV)** and **(V)** are defined as follows:
- R¹ and R³: are each independently selected from CF₂H and CF₃;
- R²: is selected from H or COOC₂H₅;
- R⁴: is selected form H, methyl, ethyl, CH₂COOCH₃, CH₂COOCH₂CH₃, phenyl;
- R⁸: is selected from ethyl, *n-, iso*-propyl, *n-,* cyclopentyl, cyclohexyl, benzyl;
- X: is F;
- R¹⁰ and R¹¹: are each independently selected from C₁-C₁₂-alkyl.

*Most preferred* is a process according to the invention, where the radicals in formula **(Ia), (Ib), (II), (III), (IV)** and **(V)** are defined as follows:
- R¹ and R³: are CF₂H,;
- R²: is H;
- R⁴: is selected from H, methyl, CH₂COOCH₂CH₃, phenyl;
- R⁸: is selected from *iso*-propyl and benzyl;
- X: is F;
- R¹⁰ and R¹¹: are each independently selected from methyl and ethyl.

Surprisingly, the pyrazoles of the formula (I) can be prepared under the inventive conditions with good yields and in high purity, which means that the process according to the invention overcomes the abovementioned disadvantages of the preparation processes previously described in the prior art.

A further aspect of the present invention are compounds of formula **(III-a):** 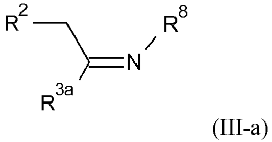 in which
- R^{3a}: is HCF₂;
- R², R⁸, R⁹: are as defined above.

*Preferred* are compounds of formula **(III-a)** in which
- R^{3a}: is HCF₂;
- R²: is H;
- R⁸: is selected from C₁-C₁₂-alkyl, C₃-C₈-cycloalkyl, benzyl.

*Mote preferred* are compounds of formula **(III-a)** in which
- R^{3a}: is HCF₂;
- R²: is H;
- R⁸: is selected from *iso*-propyl, benzyl.

A further aspect of the present invention are compounds of formula **(V-1):** 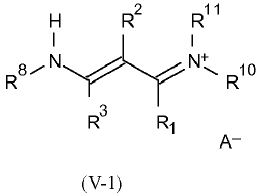 in which the radicals are as defined above.

*Preferred* are compounds of formula **(V-1)** in which
- R¹ and R³: are HCF₂;
- R²: is H;
- R⁸: is selected from C₁-C₁₂-alkyl, C₃-C₈-cycloalkyl and benzyl;
- R¹⁰ and R¹¹: are each independently selected from C₁-C₅ alkyl;
- A ⁻: is BF₄⁻.

*Mote Preferred* are compounds of formula **(V-1)** in which
- R¹ and R³: are HCF₂;
- R²: is H;
- R⁸: is selected from *iso*-propyl and benzyl;
- R¹⁰ and R¹¹: are each independently selected from methyl and ethyl;
- A⁻: is BF₄⁻.

A further aspect of the present invention are compounds of formula (V-2): 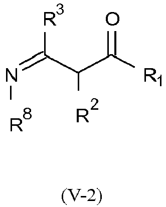 in which the radicals are as defined above.

*Preferred* are compounds of formula (V-2) in which
- R¹ and R³: are HCF₂;
- R²: is H;
- R⁸: is selected from C₁-C₁₂-alkyl, C₃-C₈-cycloalkyl and benzyl.

*Mote preferred* are compounds of formula (V-2) in which
- R¹ and R³: are HCF₂;
- R²: is H;
- R⁸: is selected from *iso*-propyl and benzyl.

A further aspect of the present invention are compounds of formula (V-3): 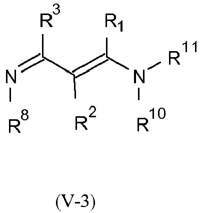 in which the radicals are as defined above.

*Preferred* are compounds of formula **(V-3)** in which
- R¹ and R³: are HCF₂;
- R²: is H;
- R⁸: is selected from C₁₋₁₂-alkyl, C₃₋₈-cycloalkyl and benzyl;
- R¹⁰ and R¹¹: are each independently selected from C₁₋₅alkyl.

*More preferred* are compounds of formula (V-3) in which
- R¹ and R³: are HCF₂;
- R²: is H;
- R⁸: is selected from *iso*-propyl and benzyl;
- R¹⁰ and R¹¹: are each independently selected from methyl and ethyl.

A further aspect of the present invention are compounds of formula **(V-4):** 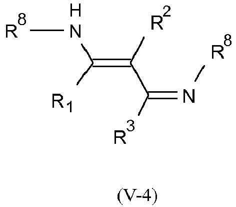 in which the radicals are as defined above.

*Preferred* are compounds of formula **(V-4)** in which
- R¹ and R³: are HCF₂;
- R²: is H;
- R⁸: is selected from C₁₋₁₂-alkyl, C₃₋₈-cycloalkyl and benzyl.

*More preferred* are compounds of formula **(V-4)** in which
- R¹ and R³: are HCF₂;
- R²: is H;
- R⁸: is selected from *iso*-propyl and benzyl.

A further aspect of the present invention are compounds of formula (V-5): 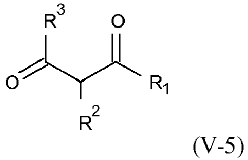 in which
- R¹ and R³: are each independently selected from HCF₂, CF₃, CF₂Cl;
- R²: is H.

### General definitions

In the context of the present invention, the term "halogen" **(Hal),** unless defined differently, comprises those elements which are selected from the group comprising fluorine, chlorine, bromine and iodine, preferably fluorine, chlorine and bromine, more preferably fluorine and chlorine.

Optionally substituted groups may be mono- or polysubstituted, where the substituents in the case of polysubstitutions may be the same or different.

**Haloalkyl:** straight-chain or branched alkyl groups having 1 to 6 and preferably 1 to 3 carbon atoms (as specified above), where some or all of the hydrogen atoms in these groups may be replaced by halogen atoms as specified above, for example (but not limited to) C₁-C₃-haloalkyl such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro,2-difluoroethyl, 2,2-dichloro-2-fluoroethyl, 2,2,2-trichloroethyl, pentafluoroethyl and 1,1,1-trifluoroprop-2-yl. This definition also applies to haloalkyl as part of a composite substituent, for example haloalkylaminoalkyl etc., unless defined elsewhere. Preference is given to alkyl groups substituted by one or more halogen atoms, for example trifluoromethyl (CF₃), difluoromethyl (CHF₂), CF₃CH₂, CF₂Cl or CF₃CCl₂.

**Alkyl** groups in the context of the present invention, unless defined differently, are linear, branched or cyclic saturated hydrocarbyl groups. The definition C₁-C₁₂-alkyl encompasses the widest range defined herein for an alkyl group. Specifically, this definition encompasses, for example, the meanings of methyl, ethyl, n-, isopropyl, n-, iso-, sec- and t-butyl, n-pentyl, n-hexyl, 1,3-dimethylbutyl, 3,3-dimethylbutyl, n-heptyl, n-nonyl, n-decyl, n-undecyl or n-dodecyl.

**Alkenyl** groups in the context of the present invention, unless defined differently, are linear, branched or cyclic hydrocarbyl groups containing at least one single unsaturation (double bond). The definition C₂-C₁₂-alkenyl encompasses the widest range defined herein for an alkenyl group. Specifically, this definition encompasses, for example, the meanings of vinyl; allyl (2-propenyl), isopropenyl (1-methylethenyl); but-1-enyl (crotyl), but-2-enyl, but-3-enyl; hex-1-enyl, hex-2-enyl, hex-3-enyl, hex-4-enyl, hex-5-enyl; hept-1-enyl, hept-2-enyl, hept-3-enyl, hept-4-enyl, hept-5-enyl, hept-6-enyl; oct-1-enyl, oct-2-enyl, oct-3-enyl, oct-4-enyl, oct-5-enyl, oct-6-enyl, oct-7-enyl; non-1-enyl, non-2-enyl, non-3-enyl, non-4-enyl, non-5-enyl, non-6-enyl, non-7-enyl, non-8-enyl; dec-1-enyl, dec-2-enyl, dec-3-enyl, dec-4-enyl, dec-5-enyl, dec-6-enyl, dec-7-enyl, dec-8-enyl, dec-9-enyl; undec-1-enyl, undec-2-enyl, undec-3-enyl, undec-4-enyl, undec-5-enyl, undec-6-enyl, undec-7-enyl, undec-8-enyl, undec-9-enyl, undec-10-enyl; dodec-1-enyl, dodec-2-enyl, dodec-3-enyl, dodec-4-enyl, dodec-5-enyl, dodec-6-enyl, dodec-7-enyl, dodec-8-enyl, dodec-9-enyl, dodec-10-enyl, dodec-11-enyl; buta-1,3-dienyl or penta-1,3-dienyl.

**Alkynyl** groups in the context of the present invention, unless defined differently, are linear, branched or cyclic hydrocarbyl groups containing at least one double unsaturation (triple bond). The definition C₂-C₁₂-alkynyl encompasses the widest range defined herein for an alkynyl group. Specifically, this definition encompasses, for example, the meanings of ethynyl (acetylenyl); prop-1-ynyl and prop-2-ynyl.

**Cycloalkyl:** monocyclic, saturated hydrocarbyl groups having 3 to 8 and preferably 3 to 6 carbon ring members, for example (but not limited to) cyclopropyl, cyclopentyl and cyclohexyl. This definition also applies to cycloalkyl as part of a composite substituent, for example cycloalkylalkyl etc., unless defined elsewhere.

**Aryl** groups in the context of the present invention, unless defined differently, are aromatic hydrocarbyl groups which may have one, two or more heteroatoms selected from O, N, P and S. The definition C₆₋₁₈-aryl encompasses the widest range defined herein for an aryl group having 5 to 18 skeleton atoms, where the carbon atoms may be exchanged for heteroatoms. Specifically, this definition encompasses, for example, the meanings of phenyl, cycloheptatrienyl, cyclooctatetraenyl, naphthyl and anthracenyl; 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl; 1-pyrrolyl, 1-pyrazolyl, 1,2,4-triazol-1-yl, 1-imidazolyl, 1,2,3-triazol-1-yl, 1,3,4-triazol-1-yl; 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

**Arylalkyl** groups (aralkyl groups) in the context of the present invention, unless defined differently, are alkyl groups which are substituted by aryl groups, may have one C₁₋₈-alkylene chain and may have, in the aryl skeleton, one or more heteroatoms selected from O, N, P and S. The definition C₇₋₁₉-aralkyl group encompasses the widest range defined herein for an arylalkyl group having a total of 7 to 19 atoms in the skeleton and alkylene chain. Specifically, this definition encompasses, for example, the meanings of benzyl and phenylethyl.

**Alkylaryl** groups (alkaryl groups) in the context of the present invention, unless defined differently, are aryl groups which are substituted by alkyl groups, may have one C₁₋₈-alkylene chain and may have, in the aryl skeleton, one or more heteroatoms selected from O, N, P and S. The definition C₇₋₁₉-alkylaryl group encompasses the widest range defined herein for an alkylaryl group having a total of 7 to 19 atoms in the skeleton and alkylene chain. Specifically, this definition encompasses, for example, the meanings of tolyl or 2,3-, 2,4-, 2,5-, 2,6-, 3,4- or 3,5-dimethylphenyl.

The term *intermediate* used in the context of the present invention describes the substances which occur in the process according to the invention and are prepared for further chemical processing and are consumed or used therein in order to be converted to another substance. The intermediates can often be isolated and intermediately stored or are used without prior isolation in the subsequent reaction step. The term "intermediate" also encompasses the generally unstable and short-lived intermediates which occur transiently in multistage reactions (staged reactions) and to which local minima in the energy profile of the reaction can be assigned.

The inventive compounds may be present as mixtures of any different isomeric forms possible, especially of stereoisomers, for example E and Z isomers, threo and erythro isomers, and optical isomers, but if appropriate also of tautomers. Both the E and the Z isomers are disclosed and claimed, as are the threo and erythro isomers, and also the optical isomers, any mixtures of these isomers, and also the possible tautomeric forms.

### Process description

The process is illustrated in Scheme 1:

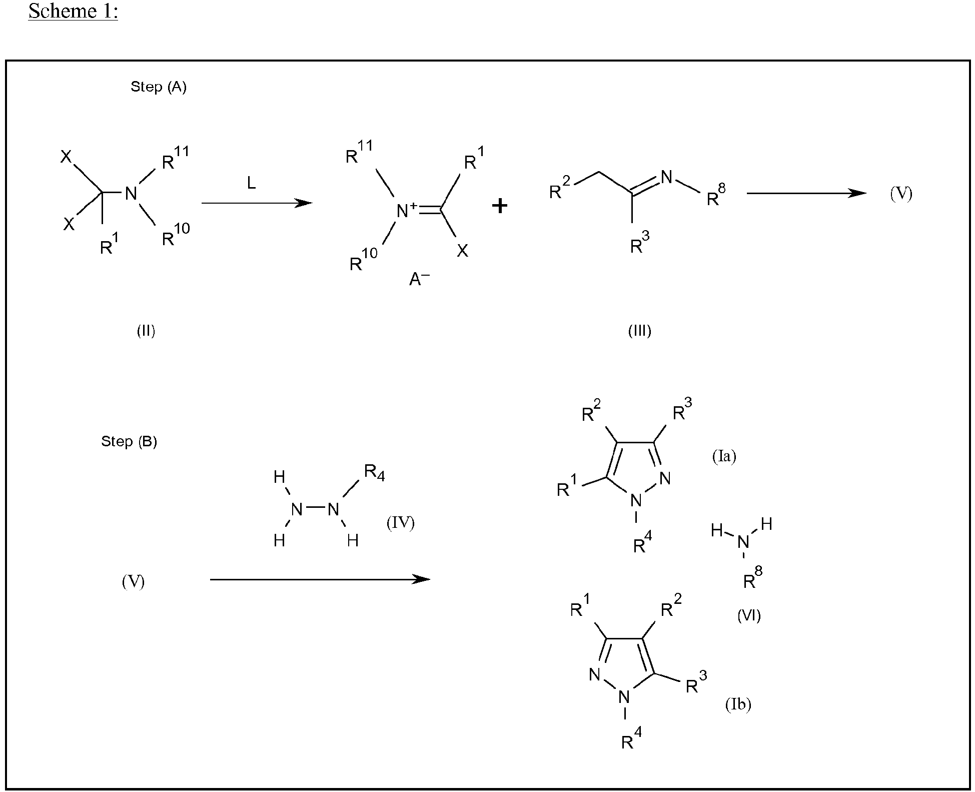

### Step (A)

In step (A), α,α-dihaloamines of the formula (II) are first reacted, in the presence of a Lewis acid [L], with compounds of the formula (III).

Preferred compounds of the general formula (II) are 1,1,2,2-tetrafluoroethyl-N,N-dimethylamine (TFEDMA), 1,1,2,2-tetrafluoroethyl-N,N-diethylamine, 1,1,2-trifluoro-2-(trifluoromethyl)ethyl-N,N-dimethylamine, 1,1,2-trifluoro-2-(trifluoromethyl)ethyl-N,N-diethylamine (Ishikawa's reagent), 1,1,2-trifluoro-2-chloroethyl-N,N-dimethylamine and 1,1,2-trifluoro-2-chloroethyl-N,N-diethylamine (Yarovenko's reagent).

Compounds of the general formula (II) are used as iminoalkylating agents. Preference is given to 1,1,2,2-tetrafluoroethyl-N,N-dimethylamine (TFEDMA) and 1,1,2,2-tetrafluoroethyl-N,N-diethylamine, and particular preference to 1,1,2,2-tetrafluoroethyl-N,N-dimethylamine. α,α-Dihaloamines such as TFEDMA and Ishikawa's reagent are commercially available or can be prepared (cf. Yarovenko et al., Zh. Obshch. Khim. 1959, 29, 2159, Chem. Abstr. 1960, 54, 9724h or Petrov et al., J. Fluor. Chem. 109 (2011) 25-31).

Yagupolskii et al. (Zh. Organicheskoi Khim. (1978), 14(12), 2493-6) shows that the reaction of Yarovenko's reagent (FClCHCF₂NEt₂) with nitriles of the formula RCH₂CN (R = CN, CO₂Et) affords the derivatives of the formula (NC)RC=C(NEt₂)CHFCl in approx. 70% yield. Compounds of the formula (III) do not react with α,α-dihaloamines of the formula (II) under this condition.

Petrov et al. (J. of Fluorine Chem. (2011), 132(12), 1198-1206) shows that TFEDMA (HCF₂CF₂NMe₂) reacts with cyclic β-diketones to transfer a difluoroacetyl group.

In a preferred embodiment of the process according to the invention, the α,α-dihaloamine is first reacted with Lewis acid [L], for example BF₃, AlCl₃, SbCl₅, SbF₅, ZnCl₂, and then the mixture of the compound of the formula (III) is added in substance or dissolved in a suitable solvent (cf. WO 2008/022777).

α,α-Dihaloamines are reacted with Lewis acids (preparation of the iminium salts of the formula (V)) according to the teaching of WO 2008/022777. According to the invention, the reaction is effected at temperatures of -20°C to +40°C, preferably at temperatures of -20°C to +30°C, more preferably at -10 to 20°C and under standard pressure. Due to the hydrolysis sensitivity of the α,α-dihaloamines, the reaction is conducted in anhydrous apparatuses under inert gas atmosphere.

The reaction time is not critical and may, according to the batch size and temperature, be selected within a range between a few minutes and several hours.

According to the invention, 1 mol of the Lewis acid [L] is reacted with equimolar amounts of the α,α-dihaloamine of the formula (II).

For the process according to the invention 1 to 2 mol, preferred 1 to1,5 mol, most preferred 1 to 1,2 mol of the α,α-dihaloamine of the formula (II) is reacted with 1 mol compound of formula (III).

Suitable solvents are, for example, aliphatic, alicyclic or aromatic hydrocarbons, for example petroleum ether, n-hexane, n-heptane, cyclohexane, methylcyclohexane, benzene, toluene, xylene or decalin, and halogenated hydrocarbons, for example chlorobenzene, dichlorobenzene, dichloromethane, chloroform, tetrachloromethane, dichloroethane or trichloroethane, ethers such as diethyl ether, diisopropyl ether, methyl tert-butyl ether, methyl tert-amyl ether, dioxane, tetrahydrofuran, 1,2-dimethoxyethane, 1,2-diethoxyethane or anisole; nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexamethylphosphoramide; sulphoxides such as dimethyl sulphoxide or sulphones such as sulpholane. Particular preference is given, for example, to THF, acetonitriles, ethers, toluene, xylene, chlorobenzene, n-hexane, cyclohexane or methylcyclohexane, and very particular preference, for example, to acetonitrile, THF, ether or dichloromethane.

The intermediates of the formula (V) formed can be used in the cyclization step without prior workup.

Especially the intermediates of the formula V-2 could be easily isolated from reaction mixture in pure form upon dilution with water. Isolated compounds of the formula V-2 are stable upon storage and react with hydrazine of the formula IV yielding the desired pyrazoles of the formula (I) in high and purity > 95-96 %, so no further purification is needed.

Alternatively, the intermediates can be isolated by suitable workup steps, characterized and optionally further purified.

Compounds of formula (III) are partially new. They can be prepared from aldehydes or ketones (VII) according to the scheme shown below, see also Roeschenthaler et al, J.Fluorine.Chem. v. 125, n. 6, 1039-1049 and Tetrahedron, 69 (2013), 3878-3884.

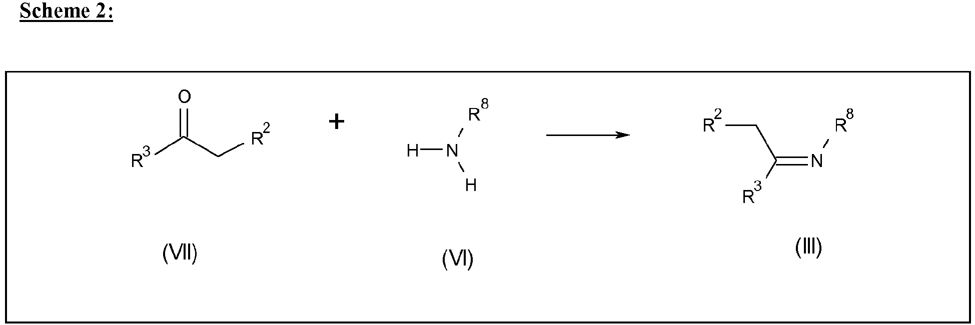

The reaction of compound (VII) and (VI) according to the invention is effected at temperatures of -40 °C to +120 °C, preferably at temperatures of +20 °C to +100 °C, more preferably at 20 °C to +60 °C and under standard pressure.

For the process according to the invention 0,9 to 2 mol, preferred 1 to 1,8 mol, most preferred 1 to 1,2 mol of the compound of the formula (VI) is reacted with 1 mol compound of the formula (VII).

In case R³ is CF₃, CF₂H, CF₂Cl and R is H it is preferable to use an excess of compound of the formula (VII), 1.02 to 2 mol, preferably 1.01 to 1,8 mol, most preferably 1.01 to 1,2 mol, of the compound of the formula (VII) for 1 mol compound of the formula (VI).

The reaction time is not critical and may, according to the batch size and temperature, be selected within a range between a few and many hours.

Suitable solvents are, for example, aliphatic, alicyclic or aromatic hydrocarbons, for example petroleum ether, n-hexane, n-heptane, cyclohexane, methylcyclohexane, benzene, toluene, xylene or decalin, and halogenated hydrocarbons, for example chlorobenzene, dichlorobenzene, dichloromethane, chloroform, tetrachloromethane, dichloroethane or trichloroethane, ethers such as diethyl ether, diisopropyl ether, methyl tert-butyl ether, methyl tert-amyl ether, dioxane, tetrahydrofuran, 1,2-dimethoxyethane, 1,2-diethoxyethane or anisole; nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexamethylphosphoramide; sulphoxides such as dimethyl sulphoxide or sulphones such as sulpholane, alkohols such as methanol, ethanol, isopropanol butanol, esters like ethyl- and propylacetate . Particular preference is given to ethylacetate, THF, acetonitriles, ethers, toluene, xylene, chlorobenzene, n-hexane, cyclohexane or methylcyclohexane, ethanol and very particular preference to ethylaacetate, toluene, acetonitrile, THF, ether, dichloromethane, ethanol. Most preferred is toluene.

### Step (B)

According to the invention, 1 mol to 2 mol, preferably 1 to 1.5 mol of the hydrazine of the formula NH₂-NHR⁴ for 1 mol of the compound of formula (V) is used.

The cyclization in step (B) of the compound of formula (V) is effected at temperatures of -40 °C to +80 °C, preferably at temperatures of +20 °C to +70 °C, more preferably at +60 °C and under standard pressure.

The reaction time is not critical and may, according to the batch size, be selected within a relatively wide range.

Typically, the cyclization step (B) is effected without changing the solvent.

Typically the cyclization of compound of the formula (V) proceeds under acidic condition.

Preference is given to mineral acids, for example H₂SO₄, HCl, HF, HBr, HI, H₃PO₄ or organic acids, for example CH3COOH, CF₃COOH, p-toluenesulphonic acid, methanesulphonic acid, trifluoromethanesulphonic acid.

According to the invention, 0.1 mol to 2 mol, preferably 0.1 to 1.5 mol of the acid for 1 mol of the compound of formula (V) is used. Suitable solvents are, for example, aliphatic, alicyclic or aromatic hydrocarbons, for example petroleum ether, n-hexane, n-heptane, cyclohexane, methylcyclohexane, benzene, toluene, xylene or decalin, and halogenated hydrocarbons, for example chlorobenzene, dichlorobenzene, dichloromethane, chloroform, tetrachloromethane, dichloroethane or trichloroethane, ethers such as diethyl ether, diisopropyl ether, methyl tert-butyl ether, methyl tert-amyl ether, dioxane, tetrahydrofuran, 1,2-dimethoxyethane, 1,2-diethoxyethane or anisole; alcohols such as methanol, ethanol, isopropanol or butanol, nitriles such as acetonitrile, propionitrile, n- or isobutyronitrile or benzonitrile; amides such as N,N-dimethylformamide, N,N-dimethylacetamide, N-methylformanilide, N-methylpyrrolidone or hexamethylphosphoramide; sulphoxides such as dimethyl sulphoxide or sulphones such as sulpholane, esters like ethyl- and propylacetate Particular preference is given, for example, to ethylacetate, acetonitrile, toluene, xylene, chlorobenzene, n-hexane, cyclohexane or methylcyclohexane, and very particular preference, for example, to ethylacetate,acetonitrile, THF, toluene or xylene. After the reaction has ended, for example, the solvents are removed and the product is isolated by filtration or distillation,, or a solution of product is first washed with water , the organic phase is concentrated under reduced pressure.

The compounds of the formula (Ia/b) where R² is COOR⁴ can then be converted to pyrazole acids of the formula (I) with R² being COOH.

Amine (VI) can be reused for the preparation of compound (III). Alternatively, it is trapped by washing the reaction mixture with acid.

The inventive compounds (Ia) and (Ib) are used for preparation of active fungicidal ingredients.

### Example 1

### N-(1,1-difluoropropan-2-ylidene)propan-2-amine, (III-1)

To the mixture of difluoracetone (94 g, 1 mol) in 500 ml methyltert.butylether (88 g., 1,5 mol) of isopropylamin was added at 10°C. After 1 h (70 g 0,5 mol) of BF₃*Et₂O was added and the mixture was stirred additionally for 1 h. Organic solution was separated from bottom syrup and solvent was distilled off at atmospheric pressure. The remaining liquid was distilled in vacuum yielding 139 g ketimine with a of b.p. 70-72°C/400 mbar.
¹H NMR (601 MHz, CDCl₃):□□: 5.83 (t, 1H), 3.74 (m, 1H), 1.92 (s, 3H), 1.15 (d, 6H) ppm.
¹⁹F (566 MHz, CDCl₃)□□□□□ -121.4 (d, 2F) ppm.

### Example 2

### N-1,1-difluoropropan-2-ylidene-1-phenylmethanamine, (III-2).

To the mixture of difluoroacetone (94 g, 1 mol) in 500 ml dichloromethane, (107 g , 1 mol) of benzylamine was slowly added at 10°C. After 6 h at 20°C CH₂Cl₂ was distilled off at reduced pressure and the remains liquid was distilled in vacuum, yielding 161 g ketimine with b.p. 80-82°C/1,3 mbar.
¹H NMR (601 MHz, CDCl₃) □□ 7.36-7.26 (m, 5H), 5.94 (t, 1H), 4.55 (s, 2H), 2.03 (s, 3H) ppm.
¹⁹F (566 MHz, CDCl₃)□□□ - -121.2 (dt, 2F) ppm.

### Example 3

### N-(1,1,1-trifluoropropan-2-ylidene)propan-2-amine, (III-3) (preparation see Example 2), b.p. 80-82°C.

### Example 4

**N-1,1,1-trifluoropropan-2-ylidene-1-phenylmethanamin** (**III**-**4**) (preparation see Example 2) b.p. 90-91 °C, 1,5 mbar.

### Example 5

### 3,5-bis(difluoromethyl)pyrazole, (I-1).

300 ml of acetonitrile were placed in a double jacketed flask and cooled to 0°C. AlCl₃ 74.4 g (0.553 mol) was added portionwise at this temperature under intensive stirring to form yellow suspension. To this suspension a solution of TFEDMA 80 g (0. 553 mol) in 350 ml acetonitrile was added at 10°C. The reaction mixture was stirred for 1 h at room temperature and solution of (53 g, 0,395 mol) of N-1,1(-difluoropropan-2-ylidene) propan-2-amine was added within 1 h at 40°C and the mixture was stirred at this temperature for 12 h. 100 ml HCl (as 5 % water solurtion) and 29 g hydrazinhydrate were added slowly to the reaction solution to keep the temperature under 40°C and the mixture was stirred for 5 h at 60°C forming two phases. The upper organic layer was separated, diluted with 500 ml methyltertbutylether, washed two times with water, dried over MgSO₄ and concentrated in vacuum to give an oily product. Vacuum destillation at 92-95°C /1 mbar gave 56,4 g (85 %) of pure 3,5-bis(difluoromethyl)-1*H*-pyrazole b) as a white solid with a of m.p. 70-71°C.
¹H NMR (601 MHz, CDCl₃) □ 11.93 (br, 1H), 6.88 (t, 2H, *J =* 54.8 Hz), 6.79 (s, 1H) ppm.
¹³C NMR (151 MHz, CDCN)□□ 103.4(p) ; 111.1 (t) ; 143.6 (br) ppm.
¹⁹F NMR (566 Mhz), □□□□□112.2 (d, br) ppm..

### Example 6

### 3,5-bis(difluoromethyl)pyrazole, (I-1).

BF₃ (247 g 0.553 mol) as 15 % Solution in CH₃CN₃ was placed in the flask and a solution of TFEDMA 80 g (0. 553 mol) in 350 ml acetonitrile was added at 10°C portionwise at this temperature under intensive stirring. The reaction mixture was stirred for 1 h at room temperature and solution of (53 g, 0,395 mol) of N-1,1(-difluoropropan-2-ylidene) propan-2-amine was added within 1 h at 40°C and the mixture was stirred at this temperature for 12 h. 20 ml HCl and 29 g hydrazinhydrate were added slowly to the reaction mixture to keep the temperature under 40°C and the mixture was stirred for 5 h at 60°C. Volitiles were removed in vacuum, 300 ml methyltertbutylether was added to the residues and organic solution was washed two times with water, dried over MgSO₄ and concentrated in vakuum to give an oily product. Vacuum destillation at 92-95°C /1 mbar gave 58 g (87 %) of pure 3,5-bis(difluoromethyl)-1*H-*pyrazole as a white solid with m.p. 70-71°C.
¹H NMR (601 MHz, CDCl₃) □ 11.93 (br, 1H), 6.88 (t, 2H, *J* = 54.8 Hz), 6.79 (s, 1H).

### Example 7

### 3-(difluoromethyl)-5-(trifluoromethyl)-1H-pyrazole, (I-2).

30 ml of acetonitrile were placed in a double jacketed flask and cooled to 0°C. BF_{3*}Et₂O 4.8 g (0.055 mol) was added portionwise at this temperature under intensive stirring to form yellow solution. A solution of TFEDMA 8 g (0.0 55 mol) in 35 ml acetonitrile was added at 10°C. and reaction mixture was stirred for 1 h at room temperature. A solution of (5, 3 g, 0,395 mol) of N-(1,1,1-trifluoropropan-2-ylidene]propan-2-amine was added within 1 h at 40°C and the mixture was stirred at 40°C for 12 h. 15 ml HCl (as 5 % water solution) and 2,9 g hydrazinhydrate was added slowly to the reaction solution to keep the temperature under 40°C and the reaction mixture was stirred for 5 h at 60°C. Water (10 mL) was added and the solution was extracted with methyltertbutyletherr (3 x 20 mL). The combined organic extracts were washed with brine (15 mL), dried over Na₂SO₄ and evaporated under reduced pressure. The crude material was purified by column chromatography on silica gel with pentane/diethyl ether (100:0 to 60:40) as eluent to afford the pure title compound (6,2 g , 85%) as a pale yellow solid.
¹H NMR (400 MHz, CDCl₃) □ 12.6 (br, 1H), 6.81 (s, 1H), 6.76 (t, 1H, *J* = 54.5 Hz) ; ¹³C (101 MHz, CDCl₃)□□ 140.7, 128.8, 120.3 (q, *J*_{C-F} = 266 Hz), 108.5 (t, *J*_{C-F} = 237 Hz), 103.8 ; ¹⁹F (376 MHz, CDCl₃)□□ -61.7 (s, 3F), -112.9 (d, 2F, *J* = 54.7 Hz) ; HRMS (ESI) calculated for C₅H₄F₅N₂ [M+H]⁺ 187.029, found 187.029.

### Example 8

### (3E/Z)-4-(benzylamino)-1,1,5,5-tetrafluoro-N,N-dimethylpent-3-en-2-iminium-tetrafluoroborate, (V-1-1).

BF₃ (2,47 g 0.0553 mol) as 15 % solution in CH₃CN₃ was placed in the flask and a solution of TFEDMA 8 g (0.0 553 mol) in 35 ml acetonitrile was added at 10°C portionwise at this temperature under intensive stirring. The reaction mixture was stirred for 1 h at room temperature and solution of (10.1 g, 0,0553 mol) of N-1,1-difluoropropan-2-ylidene-1-phenylmethanamine was added within 1 h at 40°C and the mixture was stirred at this temperature for 12 h. The solvent was removed in vakuum 2 mbar. The oily product was anylzed via NMR spectroscopy showing pure compound.

### ¹³C-NMR-Spectra

The ¹³C NMR data were taken from HSQC and HMBC spectra. The data are referenced to CD₃CN (1.3 ppm).
¹³C NMR (151 MHz, CD₃CN) □ 164.8 (s,t), 159.5 (st); 135.1 (s); 129.6 (d), 129.0 (d), 128.4 (d) ; 110.6 (dt), 109.6 (dt), 86.7 (d); 49.2 (t); 46.1 (q,br); 43.7 (q,br) ppm.
¹H NMR (601 MHz, CD₃CN) □□□□8.26 (br.s. 1H)), 7.42 (m, 2H), 7.37 (m, 3 H), 6.73 (t,1H), 6.64 (t,1H) ,5.09 (s,br, 1H), 4.58 (s, br, 2H), 3.33 (s,br., 6H) ppm.

An interaction of (3 E/Z)-4-(benzylamino)-1,1,5,5-tetrafluoro-N,N-dimethylpent-3-en-2-iminium tetrafluoroborate with N₂H₄ and HCl in Ethanol according to the conditions in example 5 gave pure 3,5-bis(difluoromethyl)pyrazole in 89 % yield.

### Example 9

### (4E-and 4Z)-benzylimino-1,1,5,5-tetrafluoro-pentan-2-one (V-2-1)

To a solution of 318 g of BF₃ (as 15.2 % w.w. solution in CH₃CN) 107 g of Tetrafluoroethyldimethylamin was added at 0 to 5°C within 40 minutes. The pale yellow solution was stirred additionally for 1 h at 0°C and the mixture was heated up to 40°C within 1 h. 122.6 g of N-benzyl-1,1-difluoro-propan-2-imine was added at 40°C so fast to maintain the temperature in the reactor between 40-45°C (addition time 45min). The reaction mixture was stirred additionally for 2 h at 40°C to give clear pale/yellow solution. Under stirring 200 ml water were added to this solution at 0°C and after 10-15 min a white precipitates starts to form. The slurry was stirred for 3-4 h at 0°C and the precipitate was filtered off, washed with 100 ml of water and dried at 40°C to give 152.2 g (yield 89 %) of the product as a white solid with a melting point of 86-87°C.
Mixture of *E*/*Z* -isomers in ratio 25:75.NMR ¹⁹F(566 MHz, CD₃CN, CFCl₃): *E*-isomer: -123.2 (d), -125.2 (d) ppm.
Z-isomer : -120.8 (d), -125.9 (d)
¹H NMR (601 MHz, CD3CN): *E*-isomer 25 %: 4.43, (d, 2H); 5.42, (s, 1H); 5.77, (t, 1H); 7.32 (t, 1H), 7.33, (m, 2H); 7.42-7.32, m, (5H); 10.67 (s, br,, 1H) ppm.
Z-isomer 75 %: 4.65, (d, 2H); 5.69, (s, 1H); 5.92, (t,1H); 6.55 (t, 1H), 5.92 (t, 1H), 7.34, (m 2H), 7.42-7.32, (m, 5H); 10.67 (s, br, 1H) ppm.

### Example 10

### (4E-and 4Z)-isopropylimino-1,1,5,5-tetrafluoro-pentan-2-one (V-2-2)

To a solution of 24.5 of BF₃ (as 15.2 % w.w. solution in CH₃CN) 8.2 g of Tetrafluoroethyldimethylamin was added at 0 to 5°C within 40 minutes. The pale yellow solution was stirred additionally for 1 h at 0°C and the mixture was heated up to 40°C within 1 h. 6.75 g of N-isopropyl-1,1-difluoro-propan-2-imine was added at 40°C so fast to maintain the temperature in reactor between 40-45°C (addition time 45min). The reaction mixture was stirred additionally for 2 h at 40°C to give clear pale/yellow solution. Under stirring 50 ml water were added to this solution at 0°C and two-phase mixture was stirred for 1 h at 0°C and the product extracted with ethylacetate, Organic extract was washed with 100 ml of water and dried over MgSO₄ to give 9,5 g (yield 89 %) of the oil as a mixture of *Z*/*E*-isomers in ratio 83:17 .
NMR ¹⁹F (566 MHz, CD₃CN): Z -isomer : -120.5 (d), -125.7 (d) ppm.
*E*-isomer: -123.1 (d), -125.0 (d) ppm.
¹H NMR (601 MHz, CD₃CN) *E*/*Z*-isomers: 1.23, 1.27 (D, 6H) ; 3.72, 3.97 (M, 1H); 5.43, 5.57 (S, 1H); 5.85, 7,33, 5.90, 6.53 (T, 1H); 10.52 (S, br, 1H) ppm.
m/z: 213.

### Example 11

### 3,5-bis(difluoromethyl)pyrazole (I-1)

2.4 g of hydrazine-hydrate were added dropwise to the suspension of 10.4 g (4*E* and 4*Z*)-benzylimino-1,1,5,5-tetrafluoro-pentan-2-one in 60 ml ethylacetate. The mixture was stirred for 1 h at room temperature and then 15.6 g of 30 % H₂SO₄ were added dropwise to reaction mixture. The slurry was stirred for 2 h at 20°C, precipitate was filtered off and organic phase washed with water. The solvent was removed in vacuum to give 6.5 g of the product with a melting point of 73-74°C.
¹H NMR (601 MHz, CDCl₃) □ 12.5 (br, 1H), 6.77 (t, 2H, *J* 54.8 Hz), 6.74 (s, 1H).

### Example 12

### N-methyl-3,5-bis(difluoromethyl)pyrazole (I-3)

To a suspension of 2,6 g of (4E and 4Z)-benzylimino-1,1,5,5-tetrafluoro-pentan-2-one in 20 ml of ethanol 0,5 g N-methylhydrazine was added at 20°C. The mixture was stirred for 1 h and then acidified by adding of 3.6 g of 30 % H₂SO₄. After 1 h the mixture was evaporated in vacuum and product extracted with methyltert.butylether. Organic phase was washed with water and concentrated in vacuum to give 1.7 g (yield 94 %) of oily product..
¹H NMR (601 MHz, CD₃CN) □ 3.95 (s, 3H), 6.77 (t, 1H), ²J=54.7 Hz); 6.78 (s, 1H); 6.95 (t, 1H, ²J=53.4 Hz) ppm.
¹⁹F NMR (566 MHz, CD₃CN, CFCl₃) □: -112.8 (d); 115.4 (d) ppm.
m/z: 182.

### Example 13

### Ethyl [3,5-bis(difluoromethyl)-1H-pyrazol-1-yl]acetate (I-4)

To a suspension of 0.7 g of (4*E* and 4*Z*)-benzylimino-1,1,5,5-tetrafluoro-pentan-2-one in 20 ml of ethanol 0,45 g of ethyl hydrazinoacetate hydrochloride was added at 20°C. The mixture was stirred for 10 h at 40°C and then acidified by adding of 0.95 g of 30 % H₂SO₄. After 1 h the mixture was evaporated in vacuum and product extracted with methyltert.butylether. Organic phase was washed with water and concentrated in vacuum to give 1.7 g (yield 94 %) of oily product.
¹H NMR (601 MHz, CD₃CN)□□: 1.24 (t, 3H), 4.20 (q, 2H), 5.07 (s, 2H) 6.79 (t, 1H, J=54.7 Hz); 6.86 (s, 1H); 6.94 (t, 1H, J=53.5 Hz) ppm.
¹⁹F NMR (566 MHz, CD₃CN) □: -112.7 (d); -115.4 (d) ppm.
m/z: 254

### Example 14 (to demonstrate the use of pyrazoles for the preparation of fungicides)

### 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate

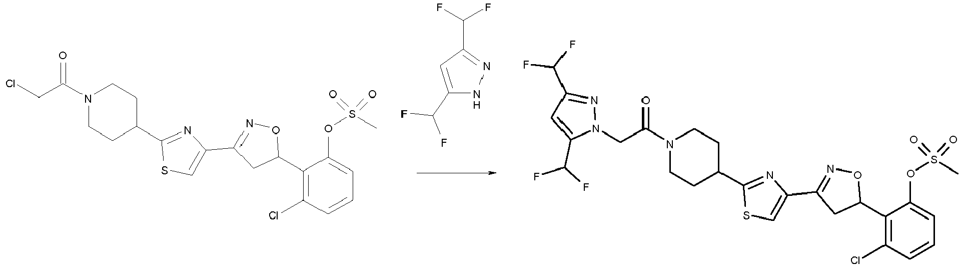

16g (0.03mol) of of 3-chloro-2-(3-{2-[1-(chloroacetyl)piperidin-4-yl]-1,3-thiazol-4-yl}-4,5-dihydro-1,2-oxazol-5-yl)phenyl methanesulfonate, 5,7g (0.033mol) 3,5-bis(difluoromethyl)-1H-pyrazole, 4.9g (0.046mol) sodium carbonate and 1.5g (0.005mol) tetrabutylammonium bromide are suspended in 100 ml acetonitrile. The mixture is heated up to 70°C and stirred for 3.5 hours. At 40°C most of the solvent is distilled off in vacuum and replaced by 100ml of toluene. The mixture is cooled to 20°C, stirred for 1 hour, seeded and then cooled to 5°C and stirred for 1 hour. A mixture of 20 ml of water and 6 ml 20% HCl is added and stirred for 30 minutes. The solid is filtered off, washed with toluene and water and dried at 45°C in vacuum.
18g of 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate with a purity of 94% is received (yield: 84%).

## Claims

1. Process for preparing 3,5-bis(haloalkyl)pyrazoles of the formula **(Ia)** and (Ib) 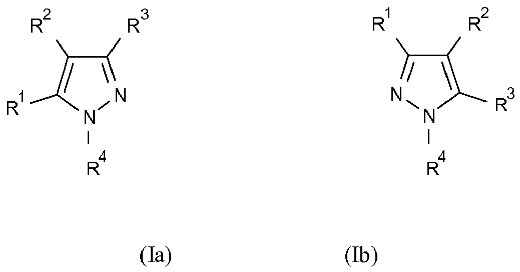 in which
R¹ and R³ are each independently selected from C₁-C₆-haloalkyl;
R² is selected from H, halogen, COOH, (C=O)OR⁵, CN and (C=O)NR⁶R⁷;
R⁴ is selected from H, C₁-C₈-alkyl, CH₂COOC₁-C₈-alkyl, aryl, pyridyl;
R⁵ is selected from C₁-C₁₂-alkyl, C₃-C₈-cycloalkyl, C₆-C₁₈-aryl, C₇-C₁₉-arylalkyl and C₇-C₁₉-alkylaryl;
R⁶ and R⁷ are each independently selected from C₁-C₁₂-alkyl, C₃-C₈-cycloalkyl, C₆-C₁₈-aryl, C₇-C₁₉-arylalkyl and C₇-C₁₉-alkylaryl or where
R⁶ and R⁷ together with the nitrogen atom to which they are bonded may form a four-, five- or six-membered ring
**characterized in that** in step (A), α,α-dihaloamines of the formula **(II),** 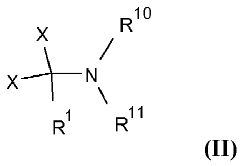 in which
R¹ is as defined above;
X is independently selected from F, Cl or Br,
R¹⁰ and R¹¹ are each independently selected from C₁-C₁₂-alkyl, C₃-C₈-cycloalkyl, C₆-C₁₈-aryl, C₇-C₁₉-arylalkyl and C₇-C₁₉-alkylaryl or where
R¹⁰ and R¹¹ together with the nitrogen atom to which they are bonded may form a five- or six-membered ring;
are reacted with compounds of the formula **(III),** 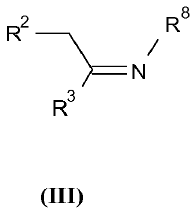 in which
R⁸ is selected from C₁-C₁₂-alkyl, C₃-C₈-cycloalkyl, C₆-C₁₈-aryl, C₇-C₁₉-arylalkyl and C₇-C₁₉-alkylaryl, OR⁹;
R⁹ is selected from C₁-C₁₂-alkyl, C₃-C₈-cycloalkyl, C₆-C₁₈-aryl, C₇-C₁₉-arylalkyl, C₇-C₁₉-alkylaryl;
R² and R³ are as defined above;
to form the compound of formula **(V): (V-1), (V-2), (V-3), (V-4) and (V-5)**
| |
|---|
| 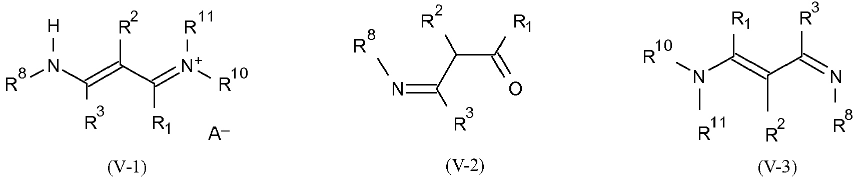 |
| 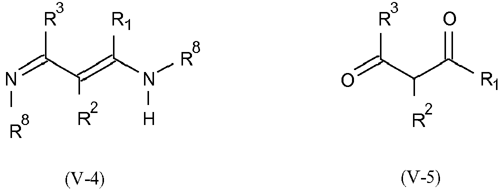 |
| A- is BF₄⁻, AlCl₃F⁻, AlF₂Cl₂⁻, AlF₃Cl⁻ or ZnCl₂F⁻ |
| R¹, R², R³, R⁵, R⁶, R⁷, R⁸, R⁹, R¹⁰, R¹¹ are as defined above |
and that in step (B) in the presence of hydrazine H₂N-NHR⁴ **(IV) -** with R⁴ being as defined above - a cyclization of **(V)** takes place to form **(Ia/Ib).**

2. Process according to Claim 1, **characterized in that**
R¹ and R³ are each independently selected from difluoromethyl, trifluoromethyl, chlorofluoromethyl, dichlorofluoromethyl, chlorodifluoromethyl, 1-fluoroethyl, 2-fluoroethyl, 2,2-difluoroethyl, 2,2,2-trifluoroethyl, 2-chloro-2-fluoroethyl, 2-difluoroethyl, 2,2-dichloro-2-fluoroethyl, 2,2,2-trichloroethyl, tetrafluoroethyl (CF₃CFH), pentafluoroethyl and 1,1,1-trifluoroprop-2-yl;
R² is selected from H, F, Cl, Br, COOCH₃, COOC₂H₅, COOC₃H₇, CN and CON(CH₃)₂, CON(C₂H₅)₂;
R⁴ is selected from H, C₁-C₈-alkyl, CH₂COOC₁-C₈-alkyl, phenyl, pyridyl;
R⁸ are each independently selected from methyl, ethyl, *n-, iso*-propyl, *n-, iso-, sec-* and *t*-butyl, cyclopropyl, cyclobutyl, cyclopentyl, cyclohexyl, phenyl, benzyl, phenylethyl, C₇-C₁₉-alkylaryl, tolyl, 2,3-, 2,4-, 2,5-, 2,6-, 3,4- or 3,5-dimethylphenyl;
X is independently selected from F or Cl;
R¹⁰ and R¹¹ are each independently selected from C₁-C₁₂-alkyl, C₃-C₈-cycloalkyl, C₇-C₁₉-arylalkyl or
R¹⁰ and R¹¹ together with the nitrogen atom to which they are bonded may form a five- membered ring.

3. Process according to Claim 1, **characterized in that**
R¹ and R³ are each independently selected from trifluoromethyl, difluoromethyl, difluorochloromethyl, pentafluoroethyl;
R² is selected from H, Cl, CN, COOC₂H₅;
R⁴ is selected from H, methyl, ethyl, *n-,* isopropyl, *n-, iso-, sec-* and *t*-butyl, *n*-pentyl, *n*-hexyl, 1,3-dimethylbutyl, 3,3-dimethylbutyl, phenyl, CH₂COOCH₃, CH₂COOCH₂CH₃;
R⁸ is selected from methyl, ethyl, *n-, iso*-propyl, *n-, iso-, sec-* und *t*-butyl, cyclopropyl, cyclobutyl, cyclopentyl, cyclohexyl, benzyl, C₇-C₁₉-alkylaryl;
X is independently selected from F or Cl;
R¹⁰ and R¹¹ are each independently selected from C₁-C₁₂-alkyl, C₃-C₈-cycloalkyl.

4. Process according to Claim 1, **characterized in that**
R¹ and R³ are each independently selected from CF₂H and CF₃;
R² is selected from H or COOC₂H₅;
R⁴ is selected form H, methyl, ethyl, CH₂COOCH₃, CH₂COOCH₂CH₃, phenyl;
R⁸ is selected from ethyl, *n-, iso*-propyl, *n-,* cyclopentyl, cyclohexyl, benzyl;
X is F;
R¹⁰ and R¹¹ are each independently selected from C₁-C₁₂-alkyl.

5. Process according to Claim 1, **characterized in that**
R¹ and R³ are CF₂H,;
R² is H;
R⁴ is selected from H, methyl, CH₂COOCH₂CH₃, phenyl;
R⁸ is selected from *iso*-propyl and benzyl;
X is F;
R¹⁰ and R¹¹ are each independently selected from methyl and ethyl.

6. Compounds of formula (V-1) 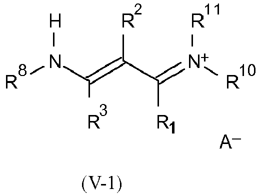 in which
R¹ and R³ are HCF₂;
R² is H;
R⁸ is selected from C₁-C₁₂-alkyl, C₃-C₈-cycloalkyl and benzyl;
R¹⁰ and R¹¹ are each independently selected from C₁-C₅ alkyl;
A⁻ is BF₄⁻.

7. Compounds of formula (V-2) 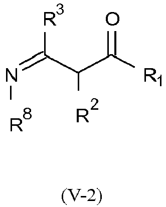 in which
R¹ and R³ are HCF₂;
R² is H;
R⁸ is selected from C₁-C₁₂-alkyl, C₃-C₈-cycloalkyl and benzyl.

8. Compounds of formula (V-3) 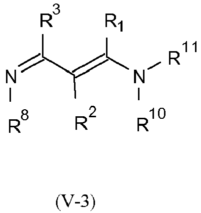 in which
R¹ and R³ are HCF₂;
R² is H;
R⁸ is selected from C₁₋₁₂-alkyl, C₃₋₈-cycloalkyl and benzyl;
R¹⁰ and R¹¹ are each independently selected from C₁₋₅ alkyl.

9. Compounds of formula (V-4) 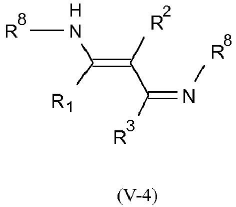 in which
R¹ and R³ are HCF₂;
R² is H;
R⁸ is selected from C₁₋₁₂-alkyl, C₃₋₈-cycloalkyl and benzyl.
